# EUROPEAN PATENT SPECIFICATION

(11) **EP 3 389 381 B1**
(45) Date of publication and mention of the grant of the patent: **15.01.2020**
(21) Application number: 16732364.1
(22) Date of filing: 27.04.2016
(51) Int. Cl.: A01N 59/12, A01N 25/10, A61K 9/08, A61K 47/02, A61K 47/10, A61K 9/00, A61K 33/18, A61K 47/34

(54) **PRODUCTION METHOD FOR A PDMS-I BASED ANTIMICROBIAL SOLUTION**
HERSTELLUNGSVERFAHREN FÜR EINE ANTIMIKROBIELLE LÖSUNG AUF BASIS VON PDMS-IOD
PROCÉDÉ DE PRODUCTION D'UNE SOLUTION ANTIMICROBIENNE À BASE DE PDMS-I

(30) Priority: 27.04.2015 TR 201505119
(43) Date of publication of application: 24.10.2018
(73) Proprietor: Çoban, Abdullah, Melikgazi/Kayseri (TR); Benk, Ayse, Kocasinan/Kayseri (TR)
(72) Inventor: Çoban, Abdullah, Melikgazi/Kayseri (TR); Benk, Ayse, Kocasinan/Kayseri (TR)
(74) Representative: Dericioglu, E. Korhan
(86) International application number: PCT/TR2016/050128
(87) International publication number: WO 2016/175728

(56) References cited:
- WO-A2-2012/007776

## Description

### Technical Area

This invention is related to production method of a PDMS-I based antimicrobial solution that serves as a barrier between the surface and environment by creating water and bacteria proof film layer on the surface it is applied and that eliminates the need to use gloves by the surgeons in the medical operations in this manner and that exhibits very high antimicrobial activity thanks to coordinated use of iodine with activated PDMS.

### Previous Technique

It was determined that iodine was a valuable medicament and superior antimicrobial with its extraordinary effective range. Human body needs this element and iodine deficiency results in goiter disease. Since a long time, iodine solutions are used as wide-spectrum bactericide agents having effect on a wide range of microorganisms such as gram positive and negative bacteria, yeast, viruses and fungi.

Iodine forms in certain oxidation situations including wholly degraded iodide (I⁻), oxidation diatomic free element (I₂), and certain high oxidation conditions combined with oxygen (for example hypo-iodate (IO⁻), iodate (IO₃⁻) and periodate (IO₄⁻)). In case of water based solutions, iodide forms the efficiently dissolving tri-iodide (I₃⁻) balance complex in the form of elemental iodine and in bonded iodine form with no antimicrobial activity. Different studies showed that iodine in free form exhibited microbial activity.

It is difficult to process iodine in its free form as it enters into chemical reaction with certain substances in and out of the body. It is also volatile and it sublimates in the atmosphere. Iodine dissolves in pure water only slightly and but if water contains iodide or iodine salt composed of poly-iodine and iodine, iodine will dissolve easier. Iodine must be in dissolved state in order that it can be used for forming useable solutions. There are many dissolution approaches and some of them are given below. First of all, Lugol's solution made in 1829 was a water based solution containing 1-5% iodine and 5-10% potassium iodine. Alcoholic solution of elemental iodine in the form of iodine tincture containing 2-7% molecular iodine was accepted as the most effective bactericide combination for a very long time. Lugol's solution and iodine tincture fell out of favor in the recent years due to the fact that they tend to cause chemical burns and tissue colorations and because of their relative instabilities, short term effects and characteristic odors. These solutions may cause swelling and bleeding of mucous membrane and they will be highly toxic when they contact with the bruises on the body. When only 0.2 ppm of iodine would be sufficient as antimicrobial on the surrounding tissue, one 1% iodine tincture solution might release extra 10000 ppm iodine powder. For that reason diluted concentrations are recommended.

The antimicrobial combinations that are most frequently used in the available technique are polyvinylpyrolydone-iodine containing iodophors and iodine tinctures. Presently, researches study to find optimal ratios of these components but they could not yet develop a superior combination that could serve as an alternative to these antimicrobial agents; that could compete with the antimicrobial agents in terms of antimicrobial activity and that have other advantages that are not normally found in these antimicrobial agents.

In the patent document numbered US20080063607 that represents known state of technique, it was shortly mentioned that iodine may be also used in silicon based foam as an anti-bacterial agent in order to keep the scope of protection as wide as possible. However there was no specific mention about the production of silicone baser foam containing iodine. Actually if they would have conducted such a study and analyzed the antimicrobial activity of the generated product, they would have also determined that silicone based material did not exhibit any antimicrobial activity with iodine unless it is activated.

Applications relating to antimicrobial solutions representing known state of technique cannot produce sufficient disinfection and are not used widely and do not bring any alternative advantages when compared with equivalent solutions except for certain repetitive benefits. A polydimethylsiloxane and iodine complex (PDMS-I) that could eliminate all of these problems is not yet developed in the known state of technique.

WO2012/007776 discloses an antiseptic formulation comprising iodine prepared in an organosiloxane solvent.

### Short Description of the Invention

Purpose of this invention is to develop a production method for a PDMS-I based antimicrobial solution composed of polydimethylsiloxane activated by sulfuric acid and iodine complex in molecular structure.

Another purpose of this invention is to develop a production method to be used to obtain transparent and stable PDMS-I based solutions with high antimicrobial activity with the use of isopropyl alcohol (IPA).

Another purpose of this invention is to develop a PDMS-I antimicrobial solution that serves as barrier between the surface and environment by forming a water proof film layer on the surface it is applied.

Another purpose of this invention is to produce a PDMS-I based antimicrobial solution that eliminates the need to use gloves by the surgeons by preventing penetration of bacteria from patient to the doctor and from doctor to the patient during surgery after it is applied onto the hands of the surgeon.

### Detailed Explanation of the Invention

In figures of the present invention of PDMS-I based antimicrobial solution production method is as follows.

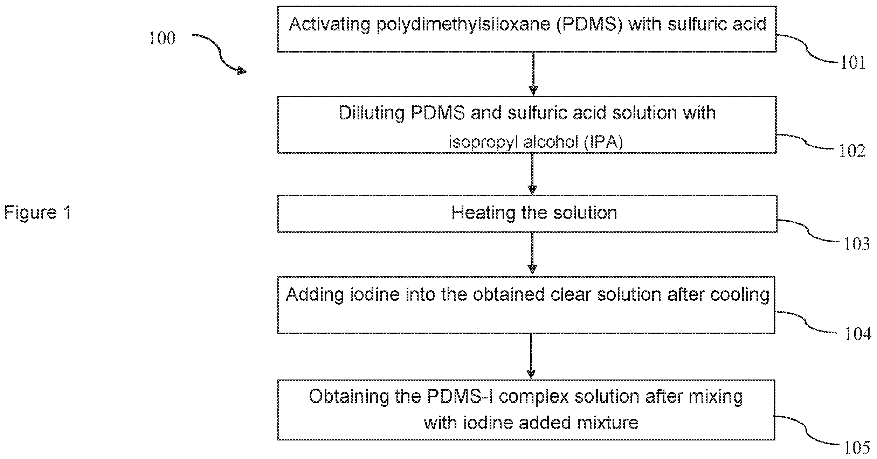

Figure 1 - Flowchart of the method of invention.
Figure 2 - Comparison table of the antimicrobial activities of control samples and samples prepared by the method of the invention.

Production method of PDMS-I based antimicrobial solution to achieve the object of the present invention (100); comprising the steps,
- Activating polydimethylsiloxane (PDMS) by sulfuric acid (101).
- Diluting PDMS and sulfuric acid solution with isopropyl alcohol (IPA) (102).
- Heating the solution (103).
- Adding iodine into clear solution obtained after cooling (104).
- Obtaining the solution formed from PDMS-I complex after the mixing of iodine added mixture (105).

In the invented method (100), first PDMS must be activated with sulfuric acid (101). This step is quite important since antimicrobial effect cannot be achieved in the complex made up of PDMS not activated by sulfuric acid. At this step, ratio of sulfuric acid to be used to dissolve PDMS must be minimum 1/10 of PDMS quantity in weight. Sulfuric acid used in the preferred application of the invention has 98% analytical purity.

PDMS solution activated by mixing with sulfuric acid is then diluted by using isopropyl alcohol (IPA) (102) and then it is heated for 10-20 minutes under reserve cooler in order that it becomes transparent (103). Transparent solution is cooled down in ambient temperature and molecular iodine (I₂) is added after controlling that there was no phase separation (104). Iodine quantity is adjusted as 1.2 units for 4.4 units of PDMS.

Then mixing is done for 30 minutes to allow iodine dissolve completely and then antimicrobial solution is obtained with the formation of PDMS-I complex (105).

In the preferred application of the invention, the solution contains in weight 4.4% PDMS, 0.44% sulfuric acid, 93-94% IPA and 1.2% molecular iodine.

With combined use of PDMS, sulfuric acid, molecular iodine and isopropyl alcohol, a surprisingly high antimicrobial activity was achieved. After that comparative analyses were conducted to observe that this activity was achieved with the coordinated use of these components only and other than that, separate use of the components or combined used of any two them or their conjugates would not yield the same result.

For that purpose, in step no 102, methyl alcohol and/or ethyl alcohol was used as solvent instead of IPA but it was observed that solutions prepared in this manner cannot become transparent and after cooling down of the solution (104), PDMS created a separate phase which was undesirable.

There is no acid that could dissolve PDMS except for strong acids like sulfuric acid and hydrofluoric acid. The reason for preferring sulfuric acid for activating PDMS (101) is that hydrofluoric acid causes skin irritation and it is much more expensive than sulfuric acid. As solvent, if we used n-pentane or hexane that easily dissolves PDMS instead of acid, solutions obtained would have much lesser antimicrobial activity as compared to the solution with sulfuric acid.

The main sample used in comparative analyses and prepared with the invented method (100) was obtained as follows:

### Main sample prepared by invented method

4.4 grams of PDMS was put into a glass balloon with bedded edges and 0.44 grams of sulfuric acid was added. Then the solution was mixed for 5 minutes in order to ensure that PDMS fully interacts with the sulfuric acid (101). Then the solution in the balloon was completed to 98.8 grams with the addition isopropyl alcohol (102) and then it was heated under reverse cooler for approximately 15 minutes (103). It was waited for the transparent solution to cool down to ambient temperature and after observing that there was no phase separation, 1.2 grams of iodine was added into it (104) and it was mixer for another 30 minutes to allow iodine to dissolve completely (105). Control samples used in comparative analyses were prepared as follows:

### Control sample 1

4.4 grams of PDMS was put into a glass balloon with bedded edges and 5 grams of n-pentane was added. Then it was mixed and dissolved under ambient conditions with the help of fish. After that, the solution was diluted to 98.8 grams with isopropyl alcohol. It was continued to mix the solution for another 10 minutes with its cover open in order to ensure that n-pentane is removed. After mixing it for another 10 minutes under reverse cooler, 1.2 grams of iodine was added to the medium and it was continued to mix for 30 minutes so that iodine completely dissolves. In this control sample, n-pentane was used instead of sulfuric acid to dissolve PDMS and effects of this difference on the antimicrobial activity were examined.

### Control sample 2

A mixture made up of 2.2 PDMS and 2.2 PVP was used instead of 4.4 PDMS in the main sample prepared with invented method in order to compare effects of PDMS and PVP on antimicrobial activity and the tried method in sample 1 was repeated as same.

### Control sample 3

PVP-I complex containing 1 - 1-2% iodine that was bought from the market and used widely (Batticon) was directly used as control sample without making any changes.

### Control sample 4

PDMS was used alone without any processing and its antimicrobial activity without other components was investigated.

### Control sample 5

0.44 grams of sulfuric acid and 99.56 grams of isopropyl alcohol were mixed and used together to determine the effect of sulfuric acid without other active substances.

### Control sample 6

Antimicrobial solution diluted with ethyl alcohol containing 4.4% PVP and 1.2% iodine was used to compare the effect of PVP and PDMS.

Then these samples were administered to different microorganisms in order to compare antimicrobial activities of the main sample and control sample.

Agar diffusion method was used to determine antimicrobial activities. In the analyses *Aeromonas hydrophila* ATCC 7965, *Bacillus cereus* RSKK 863, *Bacillus subtilis* ATCC 6633, *Escherichia coli* ATCC 25922, *Klebsiella pneumoniae* FMC 5, *Listeria monocytogenes* 1/2B, *Morganella morganii, Mycobacterium smegmatis* RUT, *Proteus mirabilis* BC 3624, *Pseudomonas aeruginosa* ATCC 27853, *Salmonella typhimurium* NRRLE 4463, *Staphylococcus aureus* ATCC 25923 (B), *Yersinia enterocolitica* ATCC 1501 bacteria and *Candida albicans* ATCC 1223 yeast were used. Each bacterium was infused into nutrient fluid media and the yeast was infused into malt extract fluid media and then diluted to contain 10⁶-10⁷ kob/ml (unit organizing colonies per millimeter) microorganisms. Each bacterium was infused 250 ml into the medium in Erlenmeyer with a temperature of 43-45°C which contained 25 ml of sterile medium (for bacteria, Mueller Hinton agar and for yeast, malt extract agar). Agars that were infused with microorganism culture were poured into Petri dishes of 9 cm and after solidification, they were waited for 1 hour in 4°C. 4 small holes with a diameter of 4 cm were opened in Petri dishes and 50 ml of each main sample and control sample was added 50 ml into these holes. *Y. enterocolitica* and C. *albicans* was left to incubate in 25°C for 24-48 hours, whereas other microorganisms were left to incubate in 35°C for 18-24 hours. All Petri dishes were examined after incubation and diameters of forming inhibition zones were measured in millimeters.

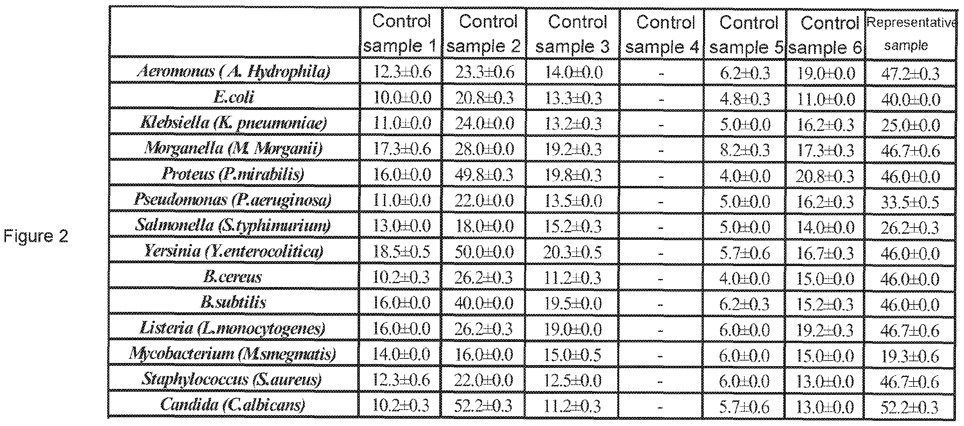

Figure 2 gives the comparison of antimicrobial activities of the main sample prepared with the invented method (100) and of the control samples.

It can be clearly seen that PDMS showed no antimicrobial activity by itself and isopropyl alcohol solution containing 0.44% sulfuric acid in weight had very low antimicrobial activity. These two results proved that very high antimicrobial activity exhibited by the main sample did not result only from PDMS or sulfuric acid or isopropyl alcohol.

If we compare the antimicrobial activities of the main sample and control sample 1, we can see that method of preparation used for the samples had quite strong effect on the antimicrobial activities of PDMS-I complexes. While the complex prepared by dissolving PDMS in an apoler solvent like n-pentane showed very low antimicrobial activity, the complex prepared by dissolving PDMS in sulfuric acid had antimicrobial effect two times stronger than the former. These results indicated that activation of PDMS by sulfuric acid was actually effective rather than single use of PDMS. That meant that correlation between PDMS and sulfuric acid was very important in preparing a complex with very high antimicrobial activity. Therefore antimicrobial activity was owed not to PDMS or sulfuric acid alone. It can be clearly seen that the structure formed while PDMS dissolved in sulfuric acid and the manner in which iodine was bonded to this structure were the basic factors determining the level of antimicrobial activity.

On the other side, it was clearly observed that antimicrobial activity of the control sample 6 with ethyl alcohol containing same quantity of 4.4% PVP did not even achieve half of the antimicrobial activity exhibited by the main sample containing 4.4% PDMS. These results indicated that PDMS was much more suitable than PVP in preparing antimicrobial complex provided that it was dissolved in sulfuric acid. Additionally, the fact that activity of control sample 2 was much lower than the antimicrobial activity of the main sample indicated that adding PVP reduced the antimicrobial activity of the complex prepared.

These important differences in antimicrobial activity could be explained to arise from bonding of iodine in inactive I₃⁻ form and from bonding of iodine in more active free iodine form in case of PDMS with sulfuric acid.

Thus it is clear that main sample containing 4.4% PDMS was much more economic than Batticon containing 8.8% PVP and used widely in the known state of technique and it had much higher antimicrobial activity.

Antimicrobial solution produced with the invented method (100) is used in especially medicine as coating material for disinfection purpose. The solution creates a disinfectant film layer on the surface it is applied and can totally prevent passage of water and bacteria between this surface and environment. Thanks to this feature of creating a film layer, the surgeons will not need to use gloves during surgery if they apply it to their hands.

In addition to the area of medicine, this solution can be used to improve lubrication characteristics of bio-diesel or bio-diesel added diesel fuels in diesel vehicles and as protective additive to prevent disintegration of bio-diesel.

## Claims

1. Production method of PDMS-I based antimicrobial solution **characterized by** the following steps (100):
- Activation of Polydimethylsiloxane (PDMS) with sulfuric acid (101),
- Diluting PDMS and sulfuric acid solution with isopropyl alcohol (IPA) (102),
- Heating the solution (103),
- Cooling down the transparent solution obtained and adding iodine (104),
- After adding iodine, mixing the solution to obtain a solution made up of PDMS-I complex (105).

2. Production method of PDMS-I based antimicrobial solution according to Claim 1 **characterized by** the following step (100): ratio of sulfuric acid used to dissolve PDMS is minimum 1/10 of PDMS quantity in weight and activation Polydimethylsiloxane (PDMS) with sulfuric acid by using sulfuric acid with an analytical purity of 98% (101).

3. Production method of PDMS-I based antimicrobial solution according to Claim 1 **characterized by** the following step (100): heating the solution diluted by IPA under reverse cooler for 10-20 minutes (103).

4. Production method of PDMS-I based antimicrobial solution according to Claim 1 **characterized by** the following step (100): Cooling down the heated solution to ambient temperature and adding iodine into it if no phase separation is observed.

5. Production method of PDMS-I based antimicrobial solution according to Claim 1 **characterized by** the following step (100): adding 1.2% of molecular iodine for 4.4% of PDMS (104) in weight.

6. Production method of PDMS-I based antimicrobial solution according to Claim 1 **characterized by** the following step (100): Mixing the solution into which iodine was added until it is fully dissolved and obtaining the solutions made up of PDMS-I complex (105).

7. Production method of PDMS-I based antimicrobial solution according to Claim 1 **characterized by** the following step (100): Using 4.4% PDMS, 0.44% sulfuric acid, 93-94% IPA and 1.2% molecular iodine in weight.

8. Using the antimicrobial solution produced with a method described in any of the claims above (100) which does not allow passage of water and bacteria between the surface and environment by forming a film layer on the surface it is applied as disinfectant on the skin.

9. Using the antimicrobial solution produced with any of the methods described in claims 1 to 7 (100) on the hands of surgeons during surgery in order to serve as gloves.

## Patentansprüche

1. Herstellungsverfahren einer antimikrobiellen Lösung auf PDMS-I-Basis, **gekennzeichnet durch** die folgenden Schritte (100):
- Aktivierung von Polydimethylsiloxan (PDMS) mit Schwefelsäure (101),
- Verdünnen von PDMS und Schwefelsäurelösung mit Isopropylalkohol (IPA) (102),
- Erhitzten der Lösung (103),
- Abkühlen der erhaltenen transparenten Lösung und Zugabe von Iod (104),
- Nach Zugabe von Iod, Mischen der Lösung, um eine Lösung zu erhalten, die aus dem PDMS-I-Komplex (105) besteht.

2. Herstellungsverfahren einer antimikrobiellen Lösung auf PDMS-I-Basis nach Anspruch 1, **gekennzeichnet durch** den folgenden Schritt (100): Das Verhältnis der zur Auflösung von PDMS verwendeten Schwefelsäure beträgt mindestens 1/10 der PDMS-Menge in Gewicht und die Aktivierung von Polydimethylsiloxan (PDMS) mit Schwefelsäure unter Verwendung von Schwefelsäure mit einer analytischen Reinheit von 98% (101)

3. Herstellungsverfahren einer antimikrobiellen Lösung auf PDMS-I-Basis nach Anspruch 1, **gekennzeichnet durch** den folgenden Schritt (100): Erhitzen der durch IPA verdünnten Lösung unter Rückkühler für 10-20 Minuten (103).

4. Herstellungsverfahren einer antimikrobiellen Lösung auf PDMS-I-Basis nach Anspruch 1, **gekennzeichnet durch** den folgenden Schritt (100): Abkühlen der erhitzten Lösung auf Umgebungstemperatur und Zugabe von Iod, wenn keine Phasentrennung beobachtet wird.

5. Herstellungsverfahren einer antimikrobiellen Lösung auf PDMS-I-Basis nach Anspruch 1, **gekennzeichnet durch** den folgenden Schritt (100): Zugabe von 1.2% molekularem Iod für 4.4% von PDMS (104) in Gewicht.

6. Herstellungsverfahren einer antimikrobiellen Lösung auf PDMS-I-Basis nach Anspruch 1, **gekennzeichnet durch** den folgenden Schritt (100): Mischen der Lösung, in die Iod zugegeben wurde, bis es vollständig gelöst ist, und Erhalten der Lösungen, die aus dem PDMS-I-Komplex (105) bestehen.

7. Herstellungsverfahren einer antimikrobiellen Lösung auf PDMS-I-Basis nach Anspruch 1, **gekennzeichnet durch** den folgenden Schritt (100): Verwendung von 4.4% PDMS, 0.44% Schwefelsäure, 93-94% IPA und 1.2% molekularem Iod in Gewicht.

8. Verwendung der antimikrobiellen Lösung, die mit einem Verfahren hergestellt wurde, das in einem der vorstehenden Ansprüche (100) beschrieben ist, das den Durchgang von Wasser und Bakterien zwischen Oberfläche und Umwelt durch Bildung einer Filmschicht auf der Oberfläche nicht zulässt, wird sie als Desinfektionsmittel auf die Haut aufgebracht.

9. Verwendung der antimikrobiellen Lösung, die mit einem Verfahren hergestellt wurde, der in den Ansprüchen 1 bis 7 (100) beschrieben ist, an den Händen von Chirurgen während der Operation erzeugt wurde, um als Handschuhe zu dienen.

## Revendications

1. Procédé de production d'une solution antimicrobienne à base de PDMS-I **caractérisé par** les étapes suivantes (100) :
- Activation de Polydiméthylsiloxane (PDMS) avec de l'acide sulfurique (101),
- Dilution de PDMS et de la solution d'acide sulfurique avec de l'alcool isopropylique (IPA) (102),
- Chauffage de la solution (103),
- Refroidissement de la solution transparente obtenue et addition d'iode (104),
- Après avoir ajouté de l'iode, mélange de la solution pour obtenir une solution composée d'un complexe PDMS-I (105).

2. Procédé de production d'une solution antimicrobienne à base de PDMS-I selon la revendication 1 **caractérisée par** l'étape suivante (100) : le rapport de l'acide sulfurique utilisé pour dissoudre PDMS est d'au moins 1/10 de la quantité de PDMS en poids et activation de Polydiméthylsiloxane (PDMS) avec de l'acide sulfurique en utilisant de l'acide sulfurique d'une pureté analytique de 98% (101).

3. Procédé de production d'une solution antimicrobienne à base de PDMS-I selon la revendication 1 **caractérisée par** l'étape suivante (100) : chauffage de la solution diluée par IPA sous refroidisseur inversé pendant 10-20 minutes (103).

4. Procédé de production d'une solution antimicrobienne à base de PDMS-I selon la revendication 1 **caractérisée par** l'étape suivante (100) : refroidissement de la solution chauffée à la température ambiante et ajout d'iode si aucune séparation de phase n'est observée.

5. Procédé de production d'une solution antimicrobienne à base de PDMS-I selon la revendication 1 **caractérisée par** l'étape suivante (100) : ajout de 1.2% d'iode moléculaire pour 4.4% de PDMS (104) en poids.

6. Procédé de production d'une solution antimicrobienne à base de PDMS-I selon la revendication 1 **caractérisée par** l'étape suivante (100) : mélange de la solution dans laquelle de l'iode a été ajouté jusqu'à dissolution complète et obtention des solutions constituées du complexe PDMS-I (105).

7. Procédé de production d'une solution antimicrobienne à base de PDMS-I selon la revendication 1 **caractérisée par** l'étape suivante (100) : utilisation de 4.4% de PDMS, 0.44% d'acide sulfurique, 93-94% d'IPA et 1.2% d'iode moléculaire en poids.

8. Utilisation de la solution antimicrobienne produite à l'aide d'une méthode décrite dans l'une quelconque des revendications ci-dessus (100) qui ne permet pas le passage de l'eau et des bactéries entre la surface et l'environnement en formant une couche de film sur la surface, elle est appliquée comme désinfectant sur la peau.

9. Utilisation de la solution antimicrobienne produite avec l'une des méthodes décrites dans les revendications 1 à 7 (100) sur les mains des chirurgiens pendant la chirurgie afin de servir de gants.
